Europäisches Patentamt

(19) European Patent Office

Office européen des brevets

(11) Publication number: **0 063 522**

**B1**

(12) **EUROPEAN PATENT SPECIFICATION**

(45) Date of publication of patent specification: **19.07.89**

(21) Application number: **82400664.7**

(22) Date of filing: **14.04.82**

(51) Int. Cl.⁴: **C 12 N 15/00, C 12 N 9/38, C 12 Q 1/68**

(54) **An inducible DNA replication-cell division coupling mechanism in Escherichia coli.**

(30) Priority: **14.04.81 GB 8111861**

(43) Date of publication of application:
**27.10.82 Bulletin 82/43**

(45) Publication of the grant of the patent:
**19.07.89 Bulletin 89/29**

(84) Designated Contracting States:
**BE CH DE FR GB IT LI NL**

(56) References cited:
NATURE, vol. 290, 30th April 1981, pages
797-799; O.HUISMAN et al.: "An inducible DNA
replication-cell devision coupling mechanism
in E. coli"

PROC. NATL. ACAD. SCI. USA, vol. 77, no. 5,
May 1980, pages 2819-2823; C.J.KENYON et
al.: "NDA-damaging agents stimulate gene
expression at specific loci in Escherichia coli"
PROC. NATL. ACAD. SCI. USA, vol. 76, no. 9,
September 1979, pages 4530-4533;

(73) Proprietor: **INSTITUT PASTEUR**
**25-28, rue du Docteur Roux**
**F-75724 Paris Cédex 15 (FR)**

(72) Inventor: **Hofnung, Maurice**
**144, rue Lecourbe**
**F-75015 Paris (FR)**
Inventor: **Quillardet, Philippe**
**6, rue Berthollet**
**F-75005 Paris (FR)**
Inventor: **Huisman, Olivier**
**5, Square de Port Royal**
**F-75013 Paris (FR)**
Inventor: **d'Ari, Richard**
**50, rue des Abbesses**
**F-75018 Paris (FR)**

(74) Representative: **Gutmann, Ernest et al**
**S.C. Ernest Gutmann - Yves Plasseraud 67,**
**boulevard Haussmann**
**F-75008 Paris (FR)**

Courier Press, Leamington Spa, England.

# EP 0 063 522 B1

(56) References cited:
M.J.CASADABAN et al.: "Lactose genes fused to exogenous promoters in ome step using a Mu-lac bacteriophage: In vivo probe for transcriptional control sequences"

JOURNAL OF BACTERIOLOGY, vol. 44, no. 1, October 1980, pages 185-191; O.HUISMAN et al.: "Further characterization of sfiA and sfiB mutations in Escherichia coli"

MOLEC. GEN. GENET., vol. 177, 1980, pages 629-636;O.HUISMAN et al.: "inducible sfi dependent division inhibition in Escherichia coli"

## Description

Cell division is a tightly regulated periodic process. In steady state cultures of Enterobacteriaceae division takes place at a well-defined cell mass[1] and is strictly coordinated with DNA replication [2]. In wild type *Escherichia coli* the formation of DNA-less cells is a very rare event[3], and interruptions of DNA replication cause an arrest of cell division[4-6]. The molecular bases of this replication-division coupling has been elusive but several models have been proposed. It has been suggested, for example, that the termination of a round of DNA replication may trigger a key event requisite to cell division[4-8]. A quite different model postulates the existence of a division inhibitor which prevents untimely division and whose synthesis is induced to high levels when DNA replication is perturbed[9]. The present invention establishes the existence of the latter type of replication-division coupling in *E. coli* and shows that the *sfiA* gene product is an inducible component of this division inhibition mechanism, synthesized at high levels after perturbations of DNA replication.

DNA replication-cell division coupling and the SOS response.

A large body of evidence points to the induction by DNA damaging treatments of a number of manifestations, collectively termed the SOS response (reviewed in refs 10—12). These include the appearance of new DNA repair and mutagenic activities, massive synthesis of the *recA* protein, lytic development of certain prophages in lysogenic strains and filamentous growth (division inhibition) in non-lysogens. The *recA*[+] and *lexA*[+] functions are required for the induction of all aspects of the SOS response. Mutant alleles of either locus can entirely prevent induction (*recA*[-], *lexA*[-]) or cause gratuitous expression at 42° of certain aspects of the SOS response (*tif* at the *recA* locus, *tsl* at the *lexA* locus). Incubation of *tif* or *tsl* strains at 42° results in the formation of long, multinucleate filaments. This filamentous growth, although not associated with detectable DNA damage, ultimately results in loss of viability.

The involvement of an inducible protein in division inhibition is suggested by two observations. First *tif* bacteria exposed briefly to high temperature form filaments on subsequent incubation at 30°, whereas if protein synthesis is inhibited by the presence of chloramphenicol during the heat pulse cell division is not inhibited on return to 30° and removal of chloramphenicol[13]. Second, normal division can be restored in *tsl* bacteria at 42° by the presence of the RNA polymerase inhibitor rifampicin at a concentration too low to block bulk protein synthesis[14]. If one could show that there is indeed a protein whose presence results in the inhibition of cell division and whose synthesis is induced by expression of the *tif* and *tsl* mutations and by perturbations of DNA replication as well, then this protein could justifiably be considered an inducible division inhibitor and the existence of the second mechanism of replication-division coupling mentioned above would be established. The *recA* protein, synthesized at high levels after inducing treatments, was suspected of being such an inducible division inhibitor[14]. It has been shown, however, that it has a purely regulatory role and is not involved in the actual process of division inhibition: in wild type cells recovering from UV irradiation normal division resumes in the presence of a high concentration of *recA* protein[15], and in *tsl* strains division inhibition can occur at 42° in the total absence of *recA* protein or in the presence of the low uninduced level[13].

The filamentous growth induced in *tif* or *tsl* strains at 42° is completely suppressed by *sfiA* and *sfiB* mutations, whereas other aspects of the SOS response are unaffected by these mutations[16,17]. The *sfiA* and *sfiB* loci thus define elements specifically involved in the filamentation process as mediated by expression of the *tif* and *tsl* mutations. Previous studies have shown that *sfi*-dependent division inhibition also occurs during thymine starvation[13].

In the study of *sfi*-dependent filamentation the *lon* mutation[18] has proved an invaluable tool. It greatly amplifies SOS associated division inhibition without affecting other manifestations of the SOS response. *E. coli* K12 *lon* mutants (or wild type *E. coli* B, a naturally occurring *lon* isolate) stop dividing after DNA damaging treatments too mild to block DNA replication and the cells grow into multinucleate aseptate filaments [19] identical in appearance to those formed by *tif* mutants at high temperature. Since this filamentation rapidly leads to loss of viability, *lon* strains are hypersensitive to treatments that perturb DNA replication[18] and to *tif* or *tsl* expression[16,20]. In all cases the hypersensitivity is suppressed by *sfiA* and *sfiB* mutations[16,17,21,22], indicating that *sfi*-dependent filamentation occurs after all DNA damaging treatments known to induce the SOS response.

Isolation of a *sfiA::lacZ* operon fusion.

The invention aimed at identifying the inducible element apparently involved in *tif*- and *tsl*-mediated filamentations. As mentioned above, it is not the *recA* protein. Attention was given to the *sfi* genes, whose products are indispensable for division inhibition in *tif* and *tsl* strains at 42° and in *lon* strains following mild inducing treatments. To obtain accurate quantitative measurements of the rates of *sfi* gene expression under various conditions the powerful method of operon fusion was chosen, using the phage Mu d(Ap, *lac*) to link the structural gene for β-galactosidase (*lacZ*) to the promoter of the operon whose regulation is to be studied[23], in the present case a *sfi* operon. The phage Mu d(Ap, *lac*), like its parent Mu-1, can insert its DNA at random locations in the host chromosome. Near one extremity of the phage genome are the *lacZ* and *lacY* genes, lacking a promoter. The phase genome is so constructed that, when inserted in a host operon

3

in the proper orientation, the *lac* genes of the prophage can be expressed from the adjacent bacterial promoter; their level of expression then reflects the level of expression of the bacterial operon in which the prophage is integrated. The Mu d(Ap, *lac*) prophage further confers ampicillin resistance on its host, permitting ready selection of lysogens.

To isolate Mu d(Ap, *lac*) insertions at *sfi* loci, a Sfi⁻ phenotype in a *lon* strain was selected, taking advantage of the hypersensitivity of the strain to low concentrations of nitrofurantoin[24]. Lysogenic Sfi⁻ clones were selected from a Mu d(Ap, *lac*) infected *lon* culture on plates containing ampicillin and nitrofuantoin. These were purified, checked for UV resistance (to confirm the Sfi⁻ character) and analysed genetically. All Sfi⁻ clones carried a *sfiA* mutation, cotransducible with *pyrD* by P1[16,21,22]. For most isolates the introduction by transduction of the Sfi⁺ character was invariably accompanied by loss of the Mu d(Ap, *lac*) prophage; these strains thus harboured a single Mu d(Ap, *lac*) prophage, located at the *sfiA* locus and conferring a Sfi⁻ phenotype. Strains of this type having a significant basal level of β-galactosidase were considered to have the *lac* genes fused to the *sfiA* promoter. One such fusion was used to study the inducibility of *sfiA* gene expression by DNA damaging treatments and its regulation by various genes known to control the filamentation response. This particular Mu d(Ap, *lac*) insertion, in addition to suppressing *lon*-linked filamentation, was also shown to suppress lethal filamentation in *tif* and *tsl* strains at 42°, and again this Sfi⁻ phenotype was indissociable from the presence of the Mu d(Ap, *lac*) prophage.

Inducibility of *sfiA* gene expression

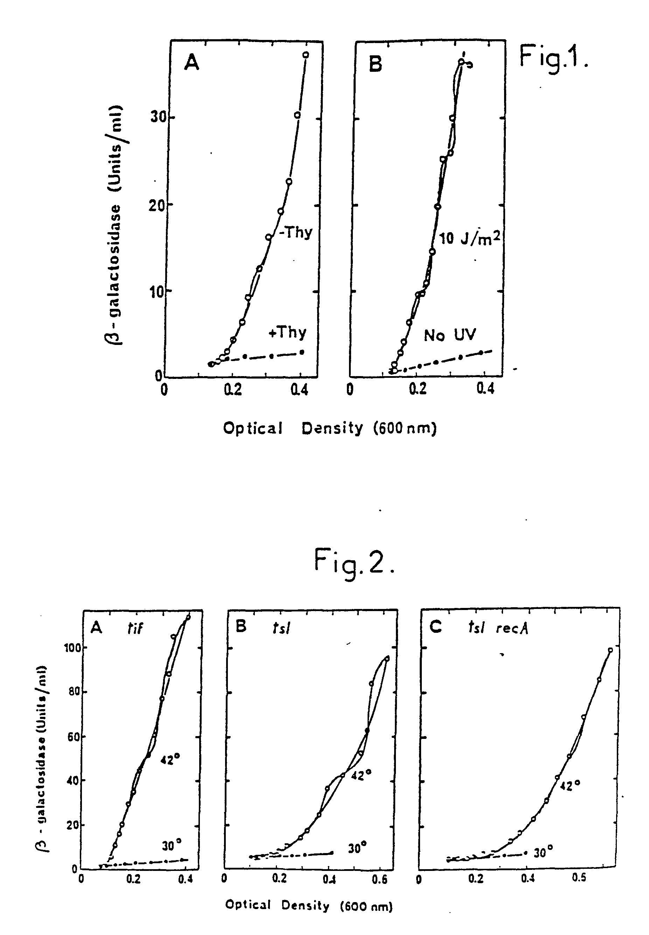

The effect of thymine starvation on *sfiA* expression was investigated in a *thyA sfiA::lacZ* fusion strain. The differential rate of β-galactosidase synthesis under these conditions quickly increases 20 to 40 fold (Figure 1A, Table 1) reaching about 0.3% of total protein synthesis. Thus, the *sfi*-dependent filamentation normally observed during thymine starvation is correlated with a large increase in the rate of *sfiA* gene expression. A dose of 10 J/m² UV radiation similarly causes a rapid 20 to 40 fold increase in the differential rate of β-galactosidase synthesis (Figure 1B, Table 1). Nalidixic acid, a specific inhibitor of DNA gyrase, and methyl methanesulfonate, a DNA alkylating agent, also induce high rates of *sfiA* gene expression (Table 1). Nitrofurantoin induces *sfi*-dependent filamentation in *lon* strains; although its primary target of action is unknown, it, too, induces a high rate of *sfiA* gene expression (Table 1). A Mu d(Ap, *lac*) prophage inserted in the *gal* operon does not lead to UV inducible β-galactosidase synthesis (Table 1), showing that the inducibility observed in the *sfiA::lacZ* strains depends on the *sfiA* location of the prophage. The conclusion derived from the above facts is that perturbations of DNA replication induce a high level of expression of the *sfiA* gene. A similar pattern of induction has been reported for the *recA* gene and the *din* genes[27,28].

Induction of λ prophage development or of mutations is widely used in tests to screen for potential carcinogens[29,30]. It is clear that *sfiA* gene expression is significantly stimulated by inducing treatments too mild to cause λ prophage induction (for example, 1 J/m² UV irradiation, 2 µg/ml nitrofurantoin). The β-galactosidase levels in appropriate *sfiA::lacZ* fusion strains thus provides a more sensitive indicator of induction of the SOS response.

Genetic regulation of *sfiA* gene expression

The effect of *sfiA* gene expression of mutations known to suppress or enhance *sfi*-dependent filamentation was studied next. *recA*⁻ and *lexA*⁻ mutations completely suppress *tif*- and *lon*-dependent filamentation [31-33]. Furthermore, cultures of *recA* or *lexA* mutant strains contain an abnormally high proportion of DNA-less cells, and this proportion increases dramatically during thymine starvation, although the DNA-containing cells in the population do filament under these conditions[3,35,36]. In *recA* or *lexA* derivatives of the *sfiA::lacZ* fusion strains no UV induction of β-galactosidase synthesis is observed in the dose range 1—10 J/m² (cf. Table 1). These same *recA* and *lexA* mutations introduced into a *gal::lacZ* fusion strain do not affect the inducibility of β-galactosidase synthesis by galactose, and the capacity of these highly UV sensitive strains to synthesize β-galactosidase is not seriously reduced by 10 J/m² UV irradiation (Table 1). The *recA*⁺ and *lexA*⁺ gene products are thus regulators of *sfiA* gene expression, most likely at the transcriptional level. Expression of the *recA* and *din* genes depends similarly on the *recA*⁺ and *lexA*⁺ gene products [28,36], and in the case of the *recA* gene, regulation has been shown to be at the transcriptional level[27,37].

The *tif* mutation in the *recA* gene causes constitutive expression of most functions at 42°[36,38], including *sfi*-dependent filamentation[16]. This expression is strongly enhanced by the presence of adenine and suppressed by the presence of guanosine plus cytidine in the medium[38,39]. In a *tif sfiA::lacZ* fusion strain a high rate of β-galactosidase synthesis is induced at 42° in the presence of adenine but not in the presence of guanosine plus cytidine (Figure 2A, Table 1). No similar induction is observed in a *tif gal::lacZ* fusion strain at 42° in the presence of adenine (Table 1).

The *tsl* mutation in the *lexA* gene causes *sfi*-dependent filamentation and massive synthesis of the *recA* protein at 42° without concomitant induction of other manifestations of the SOS response[17,36,40]. In a *tsl sfiA::lacZ* fusion strain a high level of β-galactosidase synthesis is induced at 42° (Figure 2B, Table 1). Furthermore, induction by expression of the *tsl* mutation, unlike all other inducing treatments known, does not depend on *recA*⁺ activity : in *tsl* strains carrying the *recA1* missense mutation filamentation still occurs at 42°[41] and large quantities of mutant *recA* protein are synthesized[36]. In a *tsl recA1 sfiA::lacZ* fusion strain a high level of β-galactosidase synthesis is still observed at 42° (Figure 2C, Table 1).

It has been shown that *tsl*-mediated filamentation can be suppressed by the presence in the growth medium of 4 µg/ml of the RNA polymerase inhibitor rifampicin, although this concentration has only a slight inhibitory effect on total protein synthesis[15]. This suggests that the synthesis of some component of the filamentation response is abnormally sensitive to rifampicin inhibition. In the *sfiA::lacZ* fusion strain β-galactosidase synthesis, normally well induced by 0.25% methyl methanesulfonate, was 69% inhibited by 4 µg/ml rifampicin whereas total protein synthesis was inhibited only 12%. Thus transcription of the *sfiA* gene is more sensitive to rifampicin inhibition than that of the "average" *e. coli* gene.

The *sfiB* mutations completely suppress *tif-*, *tsl-* and *lon*-dependent filamentation[16,17,21,22]. A *sfiB* mutation, however, in contrast to the *recA* and *lexA* mutations, has no inhibitory effect on *tif*-mediated or nalidixic acid induced *sfiA* gene expression (date not shown). The *sfiB* gene product is thus unlikely to be a regulator of *sfiA* gene expression. It may be involved in the action of the *sfiA* gene product, being either a second inducible component of the division inhibition mechanism or the constitutively expressed target site at which septation is inhibited. The fact that at least one *sfiB* mutation results in a 6% growth disadvantage[17] suggests constitutive expression.

All the above experiments were carried out in genetically *sfiA⁻* strains, indicating that the *sfiA* gene product, unlike the *recA* protein, is not needed for its own induction.

The *lon* mutation amplifies *sfi*-dependent filamentation, sensitizing the cell to small doses of DNA damaging treatments. The degree of sensitization depends strongly on the growth medium, being most severe in complex media[18]. At least seven different enzymes involved in cell wall biosynthesis are present at high levels in *lon* strains than in *lon⁺* strains[42], and in one case, that of the *gal* operon, the *lon* mutant has been reported to have increased transcription rates[43]. To test whether the sensitization to filamentation reflects a higher level of expression of the *sfiA* gene in *lon* strains, the induction of *sfiA* gene expression by UV irradiation was compared in *lon* and *lon⁺* *sfiA::lacZ* fusion strains growing in complex medium. No difference in basal level or induction kinetics was revealed between the two strains (*cf*. Table 1). Thus, the *lon⁺* function does not seem to be a transcriptional regulator of the *sfiA* operon. Since *lon* mutants are also deficient in the Deg protease activity[44], it is tempting to speculate that this protease is normally involved in eliminating excess *sfiA* protein, permitting a rapid resumption of cell division as soon as normal DNA replication is restored.

A DNA replication-cell division coupling mechanism.

The regulation of the SOS response is fairly well understood, at least in the case of prophage induction. DNA damaging treatments of λ lysogens result in proteolytic cleavage of the λ repressor[45]. The same cleavage can be produced *in vitro* by purified *recA* protein[46]. Since the presence in a cell of *recA* protein, even at high concentrations, is not in itself sufficient to cause induction of λ, several authors have hypothesized that DNA damaging treatments may result in the formation of signal molecule which activates the *recA* protein to its protease form[47-49]. The nature and origin of such a signal molecule, however, remain speculative. It was previously shown that induction of the SOS response by expression of the *tif* mutation involves the DNA replication complex since the induction can be suppressed by modifications of the *dnaB* protein[50]; this suggests that the replication complex may be involved in the generation of the putative signal molecule. The induction of φ80 prophage by a variety of treatments has been shown to occur simultaneously with DNA degradation at the replication fork, suggesting that the hypothetical signal molecule may be single-stranded DNA or oligonucleotides, produced by this degradation[51].

The *lexA* gene product may well be similar to a prophage repressor. Genetic evidence indicates that the *lexA* protein is a negative regulatory element governing the SOS response. The mutations called *lexA⁻* modify the protein in such a way as to prevent induction of SOS functions, even in the presence of *lexA⁺* protein[52]; they are thus analogous to the *ind⁻* mutation in the λc1 repressor gene, which prevents repressor cleavage[45]. Recent biochemical evidence has shown that the *lexA⁺* protein like the λ repressor, is cleaved *in vivo* and *in vitro* by activated *recA* protease, whereas a *lexA⁻* mutant protein is insensitive to this cleavage[53]. Mutations which completely inactivate the *lexA* protein have also been described. These are the *spr* mutations[54] and, presumably, the temperature sensitive *tsl* mutations[40]. Inactivation of the *lexA* protein causes high level expression of the *recA*[36,48] and *sfiA* genes and (in *sfi⁺* strains) lethal filamentation[40,55]. The simplest model accounting for these observations is that the *lexA* protein directly represses the *recA* and *sfiA* operons (and possibly others as well), although more complex regulatory circuits cannot be ruled out.

Whatever the precise regulatory mechanism may be, the results presented here show that a high rate of *sfiA* gene expression is induced very rapidly during thymine starvation or after UV irradiation (Figure 1). The *sfiA* gene thus provides a highly sensitive link coupling cell division to DNA replication by the following sequence of events. Perturbations or alterations of the DNA replication complex cause the generation of a signal molecule which activates the *recA* protein to its protease form. The *recA* protease then cleaves the *lexA* protein, leading to high level expression (depression) of the *sfiA* operon. Finally, the high concentration of *sfiA* protein thus produced results in an inhibition of cell division, which may serve to prevent the formation of DNA-less cells after minor perturbations of replication. In this sense the *sfiA* gene product can be considered an inducible division inhibitor.

5

References

1. Schaechter, M., Maaløe, O. & Kjeldgaard, N.O. *J. Gen. Microbiol. 19*, 592—606 (1958).
2. Cooper, S. & Hemstetter, C. *J. Molec. Biol. 31*, 519—540 (1968).
3. How, W. E. & Mount, D. W. *J. Bacteriol. 124*, 1113—1121 (1978).
4. Helmstetter, C. E. & Pierucci, O. *J. Bacteriol. 95*, 1627—1933 (1968).
5. Clark, D. J. *J. Bacteriol. 36*, 1214—1224 (1968).
6. Donachie, W. D. *J. Bacteriol. 100*, 260—268 (1969).
7. Jones, N. & Donachie, W. *Nature New Biol. 243*, 100—103 (1973).
8. Zaritsky, A. & Pritchard, R. *J. Bacteriol. 114*, 824—837 (1973).
9. Witkin, E. M. *Proc. Natn. Acad. Sci. U.S.A. 57*, 1275—1279 (1967).
10. Radman M. in *Molecular Mechanisms of Repair of DNA* (eds. Hanawalt, P. C. & Setlow, R. B.) 355—367 (Plenum, New York, 1975).
11. Witkin, E. M. *Bacteriol. Rev. 40*, 869—907 (1976).
12. Devoret, R. *Biochimie, 60*, 1135—1140 (1978).
13. Huisman, O., D'Ari, R. & George, J. *Molec. Gen. Genet. 17*, 629—636 (1980).
14. Satta, G. & Pardee, A. B. *J. Bacteriol. 133*, 1492—1500 (1978).
15. Darby, V. & Holland, I. B. *Molec. Gen. Genet. 176* 121—129 (1979).
16. George, J., Castellazzi, M. & Buttin, G. *Molec. Gen. Genet. 140*, 309—332 (1975).
17. Huisman, O., D'Ari, R. & George, J. *J. Bacteriol. 144* (1980), 185—191.
18. Howard-Flanders, P., Simson, E. & Theriot, L. *Genetics 49*, 237—246 (1964).
19. Adler, H. I. & Hardigree, A. A. *J. Bacteriol. 87*, 720—725 (1964).
20. Huisman, O., D'Ari, R. & George, J. *J. Bacteriol. 142*, 819—828 (1980).
21. Gayda, R. C., Yamamoto, L. T. & Markovitz, A. *J. Bacteriol. 127*, 1208—1216 (1976).
22. Johnson, B. F. *Genet. Res. 30*, 273—286 (1977).
23. Casadaban. M. J. & Cohen, S. N. *Proc. Natn. Acad. Sci. U.S.A., 76*, 4530—4533 (1979).
24. Kirby, E. P. Ruff, W. L. & Goldthwait, D. *J. Bacteriol. 111*, 447—453 (1972).
25. Miller, J. H. *Experiments in Molecular Genetics* (Cold Spring Harbor Laboratory, 1972).
26. Pardee, A. B., Jacob, F. & Monod, J. *J. Molec. Biol. 1*, 165—178 (1959).
27. Gudas, L. J. & Pardee, A. B. *Proc. Natn. Acad. Sci. U.S.A. 72*, 2330—2334 (1975); *J. Molec. Biol. 101*, 459—477 (1976).
28. Kenyon, C. J. & Walker, G. C. *Proc. Natn. Acad. Sci.* U.S.A. 77, 2819—2823 (1980).
29. Moreau, P., Bailone, A. & Devoret, R. *Proc. Natn. Acad. Sci. U.S.A. 73*, 3700—3704 (1976).
30. Ames, B. N., Durston, W. E., Yamasaki, E. & Lee, F. D. *Proc. Natn. Acad. Sci. U.S.A. 70*, 2281—2285 (1973).
31. Castellazzi, M., George, J & Buttin, G. *Molec. Gen. Genet. 119*, 153—174 (1972).
32. Green, M.H.L., Greenberg, J. & Donch, J. *Genet. Res. 14*, 159—162 (1969).
33. Donch, J., Green, M. H. L. & Greenberg, J. *J. Bacteriol. 96*, 1704—1710 (1968).
34. Inouye, M. *J. Bacteriol. 106*, 539—542 (1971).
35. Capaldo, F. N. & Barbour, S. D. *J. Molec. Biol. 91* 53—66 (1975).
36. Gudas, L. J. *J. Molec. Biol. 104*, 567—587 (1976).
37. McPartland, A., Green, L. & Echols, H. *cell 20*, 731—737 (1980).
38. Castellazzi, M., George, J. & Buttin, G. *Molec. Gen. Genet. 119*, 139—152 (1972).
39. Kirby, E. P., Jacob, F. & Goldthwait, D. A. *Proc. Natn. Acad. Sci. U.S.A. 58*, 1903—1910 (1967).
40. Mount, D. W., Walker, A. C. & Kosel, C. *J. Bacteriol. 116*, 950—956 (1973).
41. Mount, D. W. Walker, A. C. & Kosel, C. *J. Bacteriol. 121*, 1203—1207 (1975).
42. Hua, S. & Markovitz, A. *J. Bacteriol. 110*, 1089—1099 (1972).
43. Buchanon, C. E., Hua, S. Avni, H. & Markovitz, A. *J. Bacteriol. 114*, 891—893 (1973).
44. Gottesman, S. & Zipser, D. *J. Bacteriol. 133*, 844—851 (1978).
45. Roberts, J. W. & Roberts, C. W. *Proc. Natn. Acad. Sci. U.S.A. 72*, 147—151 (1975).
46. Roberts, J. W., Roberts, C. W. & Craig, N. L. *Proc. Natn. Acad. Sci. U.S.A. 75*, 4714—4718 (1978).
47. McEntee, K. *Proc. Natn. Acad. Sci. U.S.A. 74*, 5275—5279 (1977).
48. Gudas, L. J. & Mount, D. W. *Proc. Natn. Acad. Sci. U.S.A. 74*, 5280—5284 (1977).
49. Emmerson, P. T. & West, S. C. *Molec. Gen. Genet. 155*, 77—85 (1977).
50. D'Ari, R. George, J. & Huisman, O. *J. Bacteriol.* 140, 381—387 (1979).
51. Smith, C. L. & Oishi, M. *Proc. Natn. Acad. Sci. U.S.A. 75*, 1657—1661 (1978).
52. Mount, D. W. Low, K. B & Edmiston, S. J. *J. Bacteriol. 112*, 886—893 (1979).
53. Little, J. W., Edmiston, S. H. Pacelli, L. Z. & Mount, D. W. *Proc. Natn. Acad. Sci. U.S.A. 77*, 3225—3229 (1980).
54. Mount, D. W. *Proc. Natn. Acad. Sci. U.S.A. 74*, 300—304 (1977).
55. Brent, R & Ptashme M. *Proc. Natn. Acad. Sci. U.S.A. 77*, 1932—1935 (1980).

TABLE 1
Induction of *sfiA* gene expression

| Strain | Inducing treatment | β-galactosidase specific activity (U/mg dry weight) | | |
|---|---|---|---|---|
| | | uninduced | Induced 60 min | (dose) |
| *sfiA::lacZ thyA* | Thymine starvation | 50 | 410 | |
| *sfiA::lacZ* | UV irradiation | 30 | 140 / 440 | $(1\ J/m^2)$ / $(10\ J/m^2)$ |
| *sfiA::lacZ* | Nalidixic acid | 30 | 110 / 710 | $(2\ \mu g/ml)$ / $(40\ \mu g/ml)$ |
| *sfiA::lacZ* | Methyl methanesulfonate | 30 | 360 | (0.025 %) |
| *sfiA::lacZ* | Nitrofurantoin | 30 | 90 | $(2\ \mu g/ml)$ |
| *sfiA::lacZ recA* | UV irradiation | 10 | 10 / 10 | $(1\ J/m^2)$ / $(10\ J/m^2)$ |
| *sfiA::lacZ lexA* | UV irradiation | 10 | 10 / 10 | $(1\ J/m^2)$ / $(10\ J/m^2)$ |
| *sfiA::lacZ tif* | 42°+adenine +guanosine +cytidine | 80 | 700 / 50 | |
| *sfiA::lacZ tsl* | 42° | 200 | 450 | |
| *sfiA::lacZ tsl recA* | 42° | 200 | 400 | |
| *sfiA::lacZ lon* | UV irradiation | 20 | 400 | $(10\ J/m^2)$ |
| *gal::lacZ* | Galactose / UV irradiation | 10 / 10 | 50 / 10 | (0.4 %) / $(10\ J/m^2)$ |
| *gal::lacZ recA* | Galactose+UV | 10 | 25 | $(0.4\%,\ 10\ J/m^2)$ |
| *gal::lacZ lexA* | Galactose+UV | 10 | 30 | $(0.4\%,\ 10\ J/m^2)$ |
| *gal::lacZ tif* | 42°+adenine | 10 | 10 | |

Legend to Table 1

Bacteria were grown in M63 minimal salts medium[25] supplemented with glucose, casaminoacids and growth requirements (except for the experiments with *tsl* and *lon* strains, carried out in LB medium[25]). Cells were grown at 37° (except for the experiments with *tif* and *tsl* strains, grown at 30°) to an optical density. (600 nm) of about 0.4, then diluted to O.D. 0.1 (the thymine starved culture was first centrifuged and washed twice to remove exogenous thymine). One part of the culture was induced (by removal of thymine, UV irradiation, temperature shift or addition of the appropriate factors), the cultures were further incubated for 60 minutes and β-galactosidase specific activities were measured[25]. All strains carried a Mu $c^+$ prophage inserted in the *trp* operon in order to prevent thermal induction of the Mu d(Ap, *lac*) *c-ts62* prophage, and a chromosomal *lac* deletion to eliminate extraneous synthesis of β-galactosidase.

Figure legends

Figure 1. Inducibility of *sfiA* gene expression by thymine starvation and UV irradiation. Bacteria were grown at 37° in M63 minimal salts medium[25] supplemented with glucose, casaminoacids and growth requirements.

At an optical density of about 0.4, the cultures were adjusted to O.D. 0.1, a part of each was induced (A:

centrifuged and washed twice and resuspended in medium without thymine; B; irradiated with 10 J/m² UV light) and incubation was continued at 37°.

Samples were withdrawn every 5 minutes (induced cultures) or every 15 minutes (uninduced cultures) for optical density measurements and β-galactosidase assays (carried out as in ref. 25). Enzyme units were calculated as described in ref. 26.

Figure 2. Regulation of *sfiA* gene expression in *tif*(*recA*) and *tsl*(*lexA*) mutants. Bacteria were grown at 30° (A: in M63 minimal salts medium[25] supplemented with glucose, casaminoacids, growth requirements and—at 42°—adenine; B and C: in LB broth). At an optical density of about 0.4, the cultures were diluted to O.D. 0.1 in medium prewarmed to 42° (open symbols) or 30° (closed symbols) and incubation was continued.

Samples were withdrawn every 5 minutes (42° cultures) or every 15 minutes (30° cultures) for optical density measurements and β-galactosidase assays[25,26].

The invention further concerns the use of the recombinant obtained from any of the *sfi*-genes hereabove referred to and of the gene encoding for β-galactosidase for the transformation of suitable microorganism, particularly *E. coli*. The β-galactosidase gene may be replaced by any other gene coding for an enzyme liable of being dosed, when the expression of said *sfi* genes is caused to be indused, such as the enzymes encoded by the *UVR A* gene, the *rec*ᵃ gene, the *umuC* gene, etc.

The invention further concerns the use of the so transformed microorganism for the testing of the mutagenic power of substances suspected to possibly induce deterioration of cell DNA, to thereby cause the induction of the above mentioned SOS response, accordingly of the expression of said *sfi* genes. The method then comprises detecting the enhancement of the production of the dosable enzyme under the above defined conditions. The detection method of the mutagenic properties of any given substance then comprises cultivating said transformed microorganism in a medium suitable for its growth, such as the L medium when the microorganism is an *E. coli*, sequentially in the absence and then in the presence of said substance, whereby the mutagenic properties thereof, if any, will be revealed by an increase of the amount of the dosed enzyme (or hybrid protein encoded for by said recombinant).

An alternative of the method also comprises running comparatively two cultures of said transformed microorganism under identical conditions, except that the medium of one of said cultures shall be supplemented with the substance to be tested. The difference, if any, observed in the dosage results is then representative of the mutagenic power of the tested substance. Obviously the method is also applicable to the measuring of the reduction of mutagenic power that is liable of being caused by a drug principle expected to counteract the action of a reference substance having known mutagenic properties on the DNA of the cultivated cells, in the presence of both the mutagenic substance and the drug principle.

Advantageously the microorganism used, particularly *E. coli* will itself comprise mutations that will increase its sensitivity in the tests, particularly mutations affecting other genes known to be inducible in those instances where the DNA tends to be deteriorated by any substance penetrating in the cell, in order to favor the response of the *sfi* genes these mutations shall advantageously affect simultaneously the *UVR A*, *UVR B loci* and the *ADA locus*. Furthermore, it is desirable that a mutation further affects a gene, such as the *RFA* gene, which is known to code for the proteins causing the bacterial membranes to remain impervious to external substances.

Bacteria comprising such mutations singly are known and available. Bacteria comprising all these mutations simultaneously are obtainable by *in vivo* genetic recombinations methods known *per se*.

An *E. coli* comprising all of said mutations and the *sfiA::lacZ* fusion has been deposited at the C.N.C.M. of the Institut Pasteur of Paris under number I-152 on April 13, 1981.

**Claims**

1. Recombinant bacteria having a DNA comprising a *sfi* operon inducible by a mutagenic substance, which *sfi* operon contains a gene sequence coding for an enzyme, liable of being dosed inserted therein, so that the expression of said gene sequence is directed by the promoter of the *sfi* operon when said recombinant bacteria is contacted with a mutagenic substance.

2. The recombinant bacteria of Claim 1 which is derived from a *lon E. coli*.

3. The recombinant bacteria of Claim 2 or 3 wherein the *sfi* gene is the *sfiA* gene.

4. The recombinant bacteria of Claim 2 or 3 wherein the enzyme-encoding gene is the gene encoding for the beta-galactosidase.

5. The recombinant bacteria of Claim 2 or 3 wherein the gene encoding for the enzyme liable of being dosed is selected from among the *UVR A* gene, the *rec*ᵃ gene and the *umuC* gene.

6. The recombinant bacteria of anyone of Claims 2 to 5, which includes mutations affecting the *UVR A*, *UVR B loci* as well as the *ADA locus*.

7. A method for the testing of the mutagenic power of a substance suspected to possibly induce deterioration of cell DNA, which comprises contacting a culture of the recombinant bacteria of anyone of Claims 1 to 6 with said substance and detecting an enhanced production, if any, of the enzyme liable of being dosed as a consequence of the induction of a SOS response by said substance.

8. The method of Claim 7 wherein said recombinant bacteria is a *lon E. coli* including said

8

*sfi*-enzyme-encoding gene fusion and which comprises cultivating said *E. coli* in an L medium in the presence of said substance.

9. The method of Claim 7, or Claim 8, wherein said microorganism is grown sequentially in the absence and then in the presence of said substance, whereby the mutagenic power thereof, if any, will be revealed by an increase of the amount of the dosed enzyme produced by said recombinant bacteria.

10. The method of Claim 7 or of Claim 8 which comprises two cultures of said recombinant bacteria under identical conditions, except that the medium of one of said cultures is supplemented with the substance to be tested and the other is not, whereby the difference, if any, observed in the dosage results is then representative of the mutagenic power of the tested substance.

11. The method of any one of Claims 7 to 10, wherein said substance is a reference mutagen having known mutagenic properties and which comprises simultaneous contacting said culture of recombinant bacteria with a drug principle the counter-acting effect of which on said reference mutagen is sought, and detecting the reduction of the mutagenic effect of said reference mutagen as a measure of the counter-effect of said drug principles.

## Patentansprüche

1. Rekombinante Bakterien mit einer DNS, die aus einem mit einer mutagenen Substanz induzierbaren sfi Operon besteht, wobei das sfi-Operon eine darin eingebaute Gensequenz, die ein zu dosierendes Enzym kodiert, enthält, so daß die Expression der Gensequenz vom Promoter des sfi-Operons gesteuert wird, wenn die rekombinanten Bakterien mit der mutagenen Substanz in Berührung kommen.

2. Rekombinante Bakterien nach Ansprüch 1, die von einem lon E. coli erhalten werden.

3. Rekombinante Bakterien nach Anspruch 1 oder 2, wobei das sfi Gen das sfiA Gen ist.

4. Rekombinante Bakterien nach Anspruch 2 oder 3, wobei das das Enzym kodierende Gen das die Beta-Galactosidase kodierende Gen ist.

5. Rekombinante Bakterien nach Anspruch 2 oder 3, wobei das, das zu dosierende Enzyme kodierende Gen ausgewählt ist aus der Gruppe UVR A-Gen, rec$^a$-Gen und umuC-Gen.

6. Rekombinante Bakterien nach einem der Ansprüche 2 bis 5, die die UVR A-und UVR B-Loci sowie den ADA-Locus beeinflussende Mutationen umfassen.

7. Verfahren zum Testen der mutagenen Wirkung einer Substanz, die möglicherweise den Abbau der Zell-DNS induziert, das die Kontaktierung einer Kultur der rekombinanten Bakterien nach einem der Ansprüche 1 bis 6 mit der Substanz umfaßt, sowie den Nachweise einer möglichen erhöhten Produktion des zu dosierenden Enzyms als Folge der Induzierung einer SOS-Reaktion der besagten Substanz.

8. Verfahren nach Anspruch 7, wobei die rekombinanten Bakterien ein lon E. coli mit der, das sfi-Enzym kodierenden Genfusion ist, und das die Züchtung des E. coli in ein L Medium in Gegenwart von besagter Substanz umfaßt.

9. Verfahren nach Anspruch 7 oder 8, wobei der Mikroorganismus aufeinanderfolgend in Abwesenheit und dann in Gegenwart von besagter Substanz gezüchtet wird, wodurch ihre mögliche mutagene Wirkung durch den Anstieg der Menge des dosierte Enzyms, die durch die rekombinanten Bakterien produziert wird, nachgewiesen wird.

10. Verfahren nach Anspruch 7 oder 8, das zwei Kulturen der rekombinanten Bakterien unter gleichen Bedingungen umfaßt, mit Ausnahme, daß das Medium einer der Kulturen mit der zu testenden Substanz ergänzt wird, und das andere nicht, wodurch der mögliche Unterschied, der in den Dosierungsresultaten erkennbar ist, dann der mutagenen Wirkung der getesteten Substanz entspricht.

11. Verfahren nach einem der Ansprüche 7 bis 10, wobei die Substanz ein Referenzmutagen mit bekannten mutagenen Eigenschaften ist, und das die gleichzeitige Berührung der Kultur der rekombinanten Bakterien mit einem Medikamentengrundstoff, von dem die Gegenwirkung auf das Referenzmutagen gesucht wird, umfaßt, sowie den Nachweis der Reduktion der mutagenen Wirkung des Referenzmutagens als Maß für die Gegenwirkung der Medikamentengrundstoffe.

## Revendications

1. Bactérie recombinante ayant un ADN comprenant un opéron sfi inductible par une substance mutagène, lequel opéron sfi contient une séquence de gène qui y est insérée, ladite séquence de gène codant pour une enzyme susceptible d'être dosée de sorte que l'expression de ladite séquence de gène soit dirigée par le promoteur de l'opéron sfi quand ladite bactérie recombinante est mise en contact avec une substance mutagène.

2. Bactérie recombinante selon la revendication 1, qui est dérivée de *E. coli* lon.

3. Bactérie recombinante selon la revendication 1 ou 2, dans laquelle le gène *sfi* est le gène *sfiA*.

4. Bactérie recombinante selon la revendication 2 ou 3, dans laquelle le gène codant pour l'enzyme est le gène codant pour la β-galactosidase.

5. Bactérie recombinante selon la revendication 2 ou 3, dans laquelle le gène codant pour l'enzyme susceptible d'être dosée est sélectionnée parmi le gène UVR A, le gène rec$^a$ et le gène umuC.

6. Bactérie recombinante selon l'une quelconque des revendications 2 à 5, qui comprend des mutations affectant aussi bien les *loci* UVR B que le *locus* ADA.

7. Méthode pour tester le pouvoir mutagène d'une substance susceptible d'induire éventuellement une détérioration de l'ADN de la cellule, qui comprend la mise en contact d'une culture de la bactérie recombinante selon l'une quelconque des revendications 1 à 6, avec ladite substance et la détection d'une production accrue, le cas échéant, de l'enzyme susceptible d'être dosée résultant de l'induction d'une réponse SOS par ladite substance.

8. Méthode selon la revendication 7, dans laquelle ladite bactérie recombinante est *E. coli* lon comprenant ledit gène de fusion codant pour sfi-enzyme et qui comprend la mise en culture dudit *E. coli* dans un milieu L en présence de ladite substance.

9. Méthode selon la revendication 7, ou la revendication 8, dans laquelle ledit micro-organisme est cultivé successivement en absence puis en présence de ladite substance, ce par quoi son pouvoir mutagène, le cas échéant, ser a révélé par une augmentation de la quantité d'enzyme dosée produite par ladite bactérie recombinante.

10. Méthode selon la revendication 7, ou la revendication 8, qui comprend deux cultures de ladite bactérie recombinante dans des conditions identiques, à l'exception du fait que le milieu d'une desdites cultures est complétée par la substance à tester et l'autre ne l'est pas, à la suite de quoi la différence, le cas échéant, observée dans les résultats du dosage est alors représentative du pouvoir mutagène de la substance testée.

11. Méthode selon l'une quelconque des revendications 7 à 10, dans laquelle ladite substance est un mutagène de référence ayant des propriétés mutagènes connues et qui comprend simultanément la mise en contact de ladite culture de bactéries recombinantes avec un principe actif recherché pour son effet opposé sur ledit mutagène de référence, et la détection de la réduction de l'effet mutagène dudit mutagène de référence correspondant à la mesure de l'effet contraire dudit principe actif.

EP 0 063 522 B1

Fig.1.

β-galactosidase (Units/ml)

-Thy

+Thy

10 J/m²

No UV

Optical Density (600 nm)

Fig.2.

β-galactosidase (Units/ml)

A tif

B tsl

C tsl recA

42°

30°

Optical Density (600 nm)